# EUROPEAN PATENT APPLICATION

(11) **EP 4 113 540 A1**
(43) Date of publication of application: **04.01.2023**
(21) Application number: 21760171.5
(22) Date of filing: 26.02.2021
(51) Int. Cl.: G21K 1/02, A61B 6/00, G01T 1/161

(54) **GAMMA RAY DETECTOR WITH PLANAR SYMMETRY, MULTI-PINHOLE COLLIMATOR AND VARIABLE SAMPLING REGION**

(30) Priority: 28.02.2020 ES 202030173
(71) Applicant: Consejo Superior de Investigaciones Científicas (CSIC), 28006 Madrid (ES); Universitat Politècnica de València, 46022 Valencia (ES)
(72) Inventor: BENLLOCH BAVIERA, José María, 28006 Madrid (ES); ILISIE, Victor, 28006 Madrid (ES); MOLINER MARTÍNEZ, Laura, 28006 Madrid (ES)
(74) Representative: Tribalyte Ideas
(86) International application number: PCT/ES2021/070145
(87) International publication number: WO 2021/170895

(57) **Abstract**

The present invention belongs to the field of imaging systems based on gamma ray detectors, such as gamma cameras, equipped with multi-pinhole collimators. More specifically, the invention relates to a planar-symmetry device for high-sensitivity gamma ray detection, which allows real-time tomography image reconstruction with very good spatial resolution. Advantageously, the multi-pinhole collimators of the device move during data collection and/or one or more of the pinholes thereof moves independently, thereby allowing possible artifacts resulting from overlap areas of the detector to be completely eliminated.

## Description

### FIELD OF THE INVENTION

The present invention belongs to the field of imaging systems based on gamma ray detectors such as gamma cameras, equipped with multi-pinhole collimators. More specifically, the invention relates to a high-sensitivity gamma ray detection device, which allows real-time tomography image reconstruction with high spatial resolution.

### BACKGROUND OF THE INVENTION

Gamma cameras have great potential in the field of nuclear medicine, as they allow for an early diagnosis of small sized tumors. Said cameras are also highly useful in a wide range of preclinical studies (which contributes to designing more effective cancer treatments), as well as during surgical interventions, for locating tumors and/or lymph nodes prior to their extirpation.

One of the most recurrent problems with gamma cameras is their low sensitivity. This makes it necessary for doses of radiopharmaceuticals used in patients and/or animals for preclinical studies with these cameras to generally be very high, which entails the need for long exposures to radiation to obtain precise images of a region of interest (for example, during a gamma camera scan, to obtain details about the tumor structure). Also, due to its low sensitivity, an image obtained with a mini gamma camera for intrasurgical use takes approximately one minute to form. As this process must generally be repeated many times until the area of interest is found, this means that both the patient and the medical team involved must be exposed to a high dose of gamma radiation during the use of the camera. This circumstance generates, in the present technical field, the need to develop new models of high-sensitivity gamma cameras, which have reduced radiation exposure times compared to the cameras known to date.

The sensitivity of gamma cameras is measured by the ratio of emitted to detected photons. However, in general, both the sensitivity and the spatial resolution of the cameras can have different values throughout the field of view (FOV). In turn, the collimators used in these techniques are made of materials with high stopping power, i.e., high density and atomic number (mainly tungsten, lead, gold and platinum). Also, the collimators have one or more series of pinholes that allow the passage of photons according to specific paths. Thus, only a small portion of the emitted photons reaches the detector material (typically 10⁻⁴-10⁻¹²), which significantly limits sensitivity.

Increasing the size of the pinholes increases sensitivity but degrades spatial resolution. The trade-off between spatial resolution and sensitivity depends mainly on the size of the region of interest (ROI) or organs to be examined, the type of collimator, the energy of the emitted photons, the intrinsic spatial resolution of the detector and the radius of rotation (ROR) of the system.

The relevance of spatial resolution and sensitivity in gamma cameras is strongly conditioned by the specific application of their use. For example, for diagnostic studies in small animals, having good spatial resolution is generally more important than in most human applications. However, there are other studies in which sensitivity is considered more important than spatial resolution, such as in gated cardiac imaging techniques. These differences according to the different application have been responsible, in recent years, for the development of new systems and collimators suitable for use in gamma cameras.

New detector development technologies have also improved the intrinsic resolution of detectors. Thus, the decision as to which collimator to use for a given application depends mainly on the ratio of FOV to detector size, as well as the spatial resolution or sensitivity required. Some of the main collimator technologies currently used are, for example, parallel-hole collimators, collimators with converging/diverging holes, coded, adaptive or variable aperture collimators, multi-slit or slit-slat collimators, floating slat collimators, hybrid collimators, or multi-pinhole collimators, among many others. The present invention relates mainly to this latter group of collimators.

By way of example, in single-photon emission computed tomography (SPECT) equipment, the use of systems for controlled occlusion of the pinholes of a collimator with respect to the position of a detector is known. Systems for controlled occlusion of this type are disclosed, for example, in patent application EP 2482101 A1 for SPECT equipment with cylindrical symmetry (not part of the object of the present invention). However, said occlusion of the pinholes of the collimator leads to interruptions in the field of view during imaging, which prevents continuous sampling of the photons, representing a decrease in resolution, sensitivity, contrast and uniformity with respect to what would be desirable. Occlusion control systems furthermore exhibit considerable complexity from the point of view of tomography equipment implementation.

In the field of cameras with multi-pinhole collimators, another of the main problems is the occurrence of "artifacts" in the images, associated with the multiplexing phenomenon as a consequence of the overlapping areas of the radiation inside the detector, as it goes through the multiple pinholes of the collimator. If the multiplexing phenomenon is to be avoided by reducing the overlapping areas, cameras will generally show difficulty in obtaining complete tomographic images without truncation.

With the present invention, the limitations present in known multi-pinhole detectors are intended to be overcome by means of a novel gamma ray detection device, furthermore exhibiting high sensitivity and reduced radiation exposure times with respect to existing gamma cameras.

### BRIEF DESCRIPTION OF THE INVENTION

In light of the problems of the state of the art outlined in the previous section, an object of the present invention relates mainly to a high-sensitivity gamma ray detection device capable of reconstructing *in vivo* or *ex vivo* images of tumors and lymph nodes during surgical interventions, avoiding the presence of artifacts in the reconstructed images. Said device furthermore allows high-resolution images to be obtained during prolonged data collection but using a reduced radiation dose for the patient. The application thereof is also useful in diagnostic tests on animals, as well as in performing preclinical studies or in the decommissioning of nuclear power plants. The high sensitivity of the present detection device is mainly due to the use of collimators equipped with multiple pinholes, where said collimators are configured to move continuously, which allows continuous sampling to be performed, providing higher resolution, sensitivity, contrast and uniformity with respect to other known gamma cameras. Furthermore, depending on the embodiment, each pinhole of the collimator can also move independently. As a result of this capability and, as will be described in more detail below, imaging can be performed without the occurrence of artifacts associated with the multiplexing phenomenon, as a consequence of the overlapping areas of the radiation inside the detector. In addition, the device of the invention allows complete tomographic images to be obtained without truncation.

More specifically, the mentioned object of the invention is preferably performed by means of an imaging device of a radiation-emitting object from the gamma ray detection coming from said source of radiation, comprising:
- a detector equipped with one or more gamma radiation-sensitive materials;
- electronic means configured for the reading and processing of one or more signals of gamma radiation by the detector; and
- a gamma ray collimator (performed for example with lead, tungsten or the like) equipped with a plurality of pinholes (through which the gamma rays penetrate) arranged, relative to the detector, such that they provide at least one sampling region on the field of view (FOV) of the detector.

Preferably, the detector, the electronic reading and processing means and the gamma ray collimator of the device of the invention are arranged in substantially parallel respective planes, thus adopting a camera configuration with planar symmetry.

Advantageously, in said device, the position of the pinholes of the collimator is variable relative to the position of the detector in the device, such that the sampling region on the field of view (FOV) of the detector provided by one or more cones of incidence of gamma radiation upon passing through the pinholes, is modified with the variation of the positions of said pinholes with respect to the detector. Likewise, and even more preferably in the invention, the variation of the position of the pinholes of the collimator with respect to the detector takes place continuously, i.e., the pinholes travelling a relative distance with respect to the detector, but without occlusion of said pinholes along said distance while obtaining the images.

In a preferred embodiment of the invention, the collimator comprises a plurality of aperture patterns by means of covering and/or uncovering the pinholes.

In another preferred embodiment of the invention, one or more of the pinholes of the collimator are covered by corresponding plugs (preventing the passage of gamma radiation, manufactured for example with lead, tungsten or the like), the plugs being arranged on the collimator or on a support arranged thereon, where said support is equipped with pinholes adapted for housing the plugs.

In a preferred embodiment of the invention, the positions of the pinholes are independently movable with respect to one another, in the collimator.

In another preferred embodiment of the invention, the position of the collimator and/or of its pinholes exhibits relative rotational and/or translational movement with respect to the position of the detector.

In another preferred embodiment of the invention, the position of the collimator and/or of its pinholes exhibits continuous relative rotational movement with respect to the position of the detector, about an axis substantially perpendicular to the plane formed by the collimator, where said axis does not go through any of the pinholes of the collimator.

In another preferred embodiment of the invention, the axes of all the pinholes of the collimator are substantially parallel, such that the sampling region on the FOV of the detector provided by the cones of incidence of gamma radiation upon passing through the pinholes does not exhibit any overlap for at least two pinholes.

In another preferred embodiment of the invention, the device has one or more overlapping areas between the cones of incidence on at least one detection surface of the detector, where said areas are modified with the variation of the positions of the pinholes with respect to the detector. Preferably, for this configuration and for the purpose of avoiding the multiplexing phenomenon, image reconstruction will be performed first forming an initial image only with the gamma rays which are not detected in the overlapping areas. Once said prior image has been formed, and using statistical methods, gamma rays detected in the overlapping areas will also subsequently be taken into account to increase sensitivity.

In another preferred embodiment of the invention, the device does not exhibit any overlapping area between the cones of incidence on the detection surface. However, as said areas on the detection surface are modified with the variation of the positions of the pinholes with respect to the detector, the cones of detection cover the entire detection surface (except the peripheral area) during continuous movement of the collimator. This configuration is, for all intents and purposes, equivalent to having overlapping areas on the surface of the detector but knowing at all times through which pinhole a gamma ray has passed before impacting the overlapping area. The problem with multiplexing is thereby solved, directly obtaining complete (not truncated) images without artifacts due to overlap.

In another preferred embodiment of the invention, the collimator is mobile with respect to the detector, such that each of its pinholes describes a circular path around its corresponding center of rotation (different for each of the pinholes). Alternatively, the collimator is mobile with respect to the detector, such that said collimator can rotate around a fixed point in space and, more preferably, said fixed point is such that the angles of visibility of the field of view (FOV) areas of the detector, under the movement of the collimator, are variable until a complete or partial rotation of said collimator has been achieved.

In another preferred embodiment of the invention, the collimator has circular groove-shaped pinholes, and where said pinholes are partially covered by a plug with a variable position in said groove.

In another preferred embodiment, the pinholes have a cylindrical or double cone shape, and each pinhole can have a different field of view (angular aperture and inclination).

In another preferred embodiment of the invention, the pinholes of the collimator are distributed such that each of said pinholes, except those arranged in the perimetral region of the collimator, has six neighboring pinholes, located at the same distance, forming a regular hexagon; and wherein said pinholes optionally have the same angular aperture.

Another object of the invention relates to an imaging system by means of gamma ray detection, comprising one or more devices according to any of the embodiments described herein, and wherein the electronic means for the reading and processing of the signals of the detector are connected to an image reconstruction device, from the processing of said signals.

In a preferred embodiment of the invention, the mentioned system comprises a mobile platform adapted for orienting the device towards different regions of a source of gamma radiation.

In the scope of the present invention, the term substantially shall be understood to mean "identical" or comprised in a margin of variation of ±10%.

### DESCRIPTION OF THE DRAWINGS

The above and other features and advantages will be more fully understood from the detailed description of the invention and of the preferred embodiments relating to the attached figures, which are described in the following paragraphs.
Figure 1 shows a schematic depiction of a preferred embodiment of the object of the present invention relating to a gamma detection device made up of a mobile multi-pinhole collimator with planar symmetry.
Figure 2 shows a schematic depiction of an array of plugs for the gamma radiation configured to be housed in the pinholes of the plane collimator of Figure 1.
Figure 3 shows a schematic depiction of a preferred embodiment of the object of the present invention relating to a gamma detection device made up of a mobile multi-pinhole collimator with planar symmetry, where the continuous relative rotational movement between said collimator and a detector is depicted, where the axis of said rotation does not coincide with any of the axes of the pinholes of the collimator.
Figures 4A-4C show a schematic depiction of a possible multi-pinhole collimator translational movement sequence with respect to the detector of the device, in a preferred embodiment of the invention.

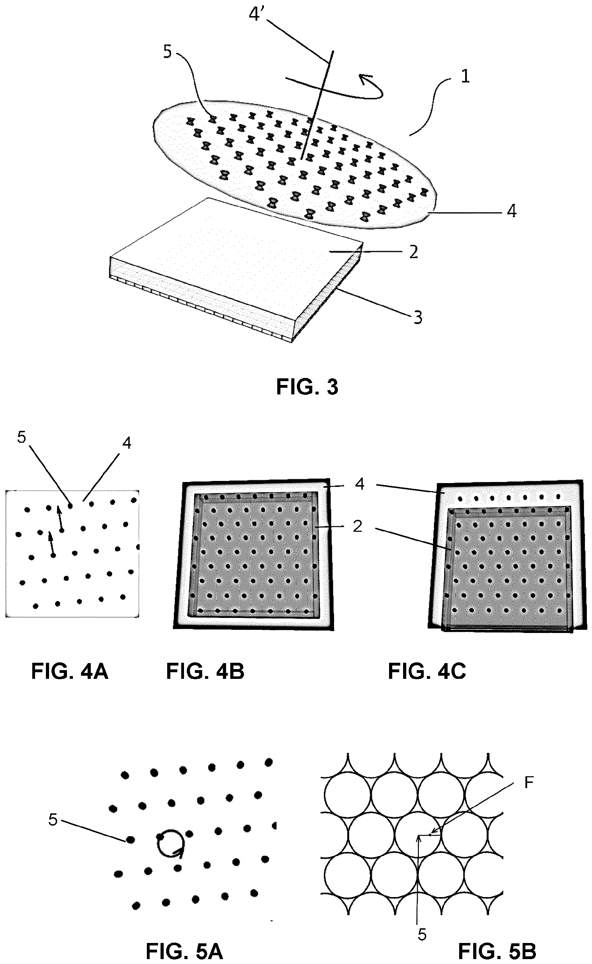

Figures 5A-5B show a depiction of a possible center of rotation for a circular movement of the collimator around a fixed point, which is positioned, for example, at ¼ of the distance between two consecutive pinholes (the circumferences depict the areas of incidence on the surface of the detector, corresponding to each pinhole). Said point of rotation allows a noncyclic sampling (until a rotation of 360° has been achieved) of the field of view. The circumferences of Figure 5B depict the bases of the cones of incidence of the gamma rays on the detection surface, which in this case do not exhibit overlap.
Figure 6 shows a schematic depiction of a collimator with circular groove-shaped, partially covered, pinholes the covered areas of which vary while collecting data.
Figure 7 shows a schematic depiction of a distribution of pinholes which is periodically repeated, where each pinhole, except the one located on the edge of the collimator, has six neighboring pinholes located at the same distance, forming a regular hexagon.
Figure 8A shows a two-dimensional schematic depiction of a gamma camera with a multi-pinhole collimator and a gamma ray detector. The area of incidence of the gamma rays, allowed by the collimator (which would correspond to cones of incidence in three dimensions) is also shown schematically. The angular aperture allows there to be overlap of the cones of incidence on the surface of the detector, which increases the sensitivity of the camera.
Figure 8B shows a schematic depiction of a possible way to cover the pinholes of a multi-pinhole collimator according to the invention, in order to temporarily prevent access of the gamma rays to the overlapping area on the detection surface.

### Reference numbers used in the drawings:

| | |
|---|---|
| (1) | Gamma ray detection device |
| (2) | Detector equipped with gamma radiation-sensitive means |
| (3) | Optical photosensors, gamma radiation detection electronics |
| (4) | Multi-pinhole collimator |
| (4') | Axis of rotation of the collimator with respect to the detector |
| (5, 5') | Pinholes |
| (6) | Plug for the pinholes |
| (7) | Support of the plugs for the pinholes |
| (8) | Gamma rays |
| (9) | Source of radiation |
| (10) | Cone of incidence of the gamma rays |
| (11) | Overlapping areas of the cones of gamma radiation |

### DETAILED DESCRIPTION OF THE INVENTION

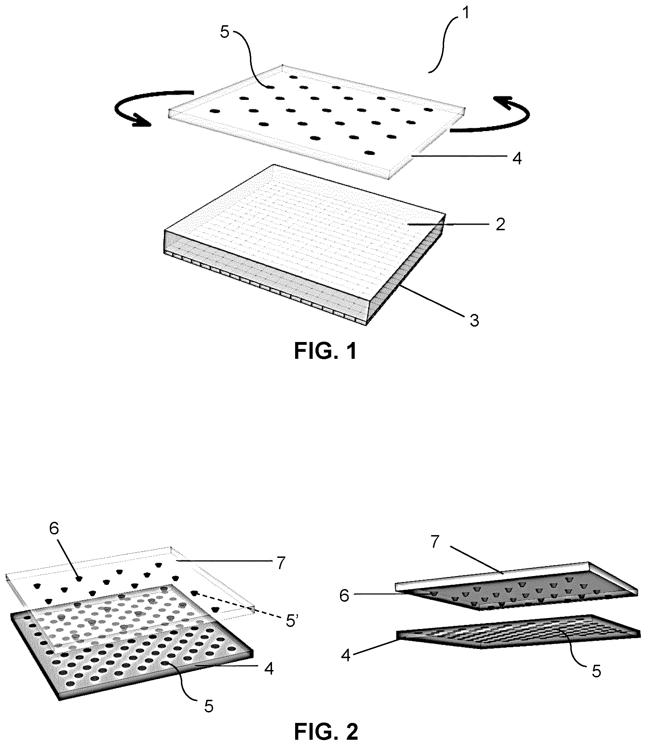

A detailed description of the invention relating to different preferred embodiments thereof based on Figures 1-8 herein is provided below. Said description is provided for purposes for illustrating but not limiting the claimed invention.

As described in the preceding sections, the imaging device (1) for imaging radiation-emitting objects from the gamma ray detection of the invention comprises at least the following essential elements (Figure 1):
- a detector (2) equipped with gamma radiation-sensitive means and a corresponding detection electronics (3), configured to calculate the energy from the interaction of the incident gamma rays on the device (1), as well as to determine the position where said interaction occurs; and
- a mobile gamma ray collimator (for example, of lead, tungsten or the like) (4), equipped with a plurality of pinholes (5) (through which the gamma rays penetrate), adapted for performing a sampling of multiple regions of the field of view (FOV) of the detector (2), which guarantees, by means of a suitable processing of the data acquired by the detector (2) (for example, through a computer connected to the detection electronics (3)), the absence of artifacts and obtaining a complete final image, without truncation.

Preferably in the device, and as shown in Figure 1, the detector (2), the electronic reading and processing means (3) and the gamma ray collimator (4) of the device of the invention are arranged in substantially parallel respective planes, thus adopting a camera configuration with planar symmetry.

In a complementary or alternative manner, the pinholes (5) are adapted in the device (1) so that they can be covered and/or uncovered, therefore equipping the collimator (4) with different configurable aperture patterns. An example of this embodiment is shown in Figure 2, where a collimator (4) is depicted with pinholes (5) which are covered by a plurality of plugs (6), formed, for example, from tungsten and/or lead. In different embodiments of the invention, said plugs (6) can be arranged directly on the collimator (4) or on a support (7) arranged thereon, where said support (7) is equipped with pinholes (5') adapted for housing the plugs (6). In any of the preceding configurations, the variation of the position of the pinholes (5) of the collimator (4) with respect to the detector (2) takes place continuously, i.e., the pinholes (5) travel a relative distance with respect to the detector (2), but without occlusion of said pinholes along said distance, except possibly by plugs placed prior to acquisition.

In another preferred embodiment of the invention, the positions of the pinholes (5) can move independently with respect to other in the collimator (4). Optionally, the detector (2) can furthermore be configured to rotate and/or move around the region of interest of the object/animal/patient in respect of which images are to be obtained. Therefore, the overlapping areas, if they exist on the detection surface of the detector (2), vary during image acquisition.

In another preferred embodiment of the invention, the position of the collimator (4) and/or of its pinholes (5) exhibits continuous relative rotational movement with respect to the position of the detector, about an axis (4') substantially perpendicular to the plane formed by the collimator (4), and where said axis (4'), even more preferably, does not go through any of the pinholes (5) of the collimator (4). Said situation is illustrated in Figure 3.

In another preferred embodiment of the invention, the axes corresponding to the pinholes (5) of the collimator (4) are substantially parallel, such that the sampling region on the FOV of the detector (2) provided by the cones of incidence of gamma radiation upon passing through the pinholes (5) does not exhibit any overlap for at least two of said pinholes (5).

As mentioned for different embodiments of the invention, the relative movement between the collimator (4) and the detector (2) is, preferably, a translational and/or rotational movement, as shown in Figures 4 and 5, respectively. The example of the translational movement (Figures 4A) consists of moving the collimator (4) with respect to the detector (2) (Figures 4B-4C), so that the pinholes (5) adopt different configurations for one and the same region of the field of view (FOV).

For the case of rotation (Figures 5A-5B), in a preferred embodiment of the invention, the collimator (4) moves in a relative manner with respect to the detector (2), such that each of its pinholes (5) describes a circular path around its corresponding center of rotation (different for each of the pinholes, Figure 5A), the final position coinciding with the initial position. In another embodiment of the circular movement (Figure 5B), the collimator (4) can rotate around a fixed point (F) in space, and with relative movement with respect to the detector (2). Said point is preferably the center of the detector (such that the axis of rotation passes through the center of the detector).

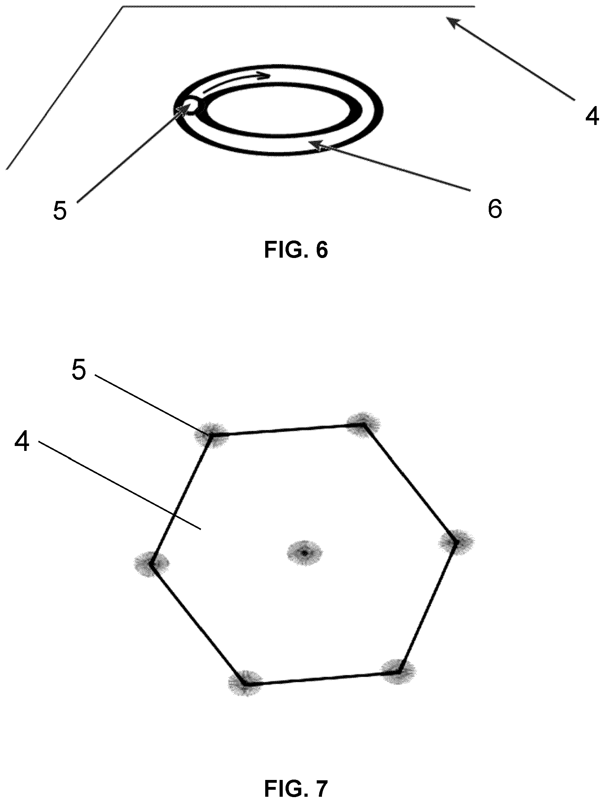

In a third preferred embodiment in the case of rotation (Figure 6), the collimator (4) has circular groove-shaped pinholes (5). Preferably, said pinholes (5) are partially covered by a plug (6) (for example, plotting a circular portion), where the covered area varies while collecting data with the device (1), such that the pinholes (5) plot a circular path in space.

In another preferred embodiment of the invention (Figure 7), the pinholes (5) of the collimator (4) are distributed such that each of said pinholes (5) (except those arranged on the edge of the collimator (4)) has six neighboring pinholes (5), located at the same distance, forming a regular hexagon. Preferably, said pinholes (5) have the same angular aperture, which ensures that each pinhole (5) allows uniform sampling to be performed during imaging.

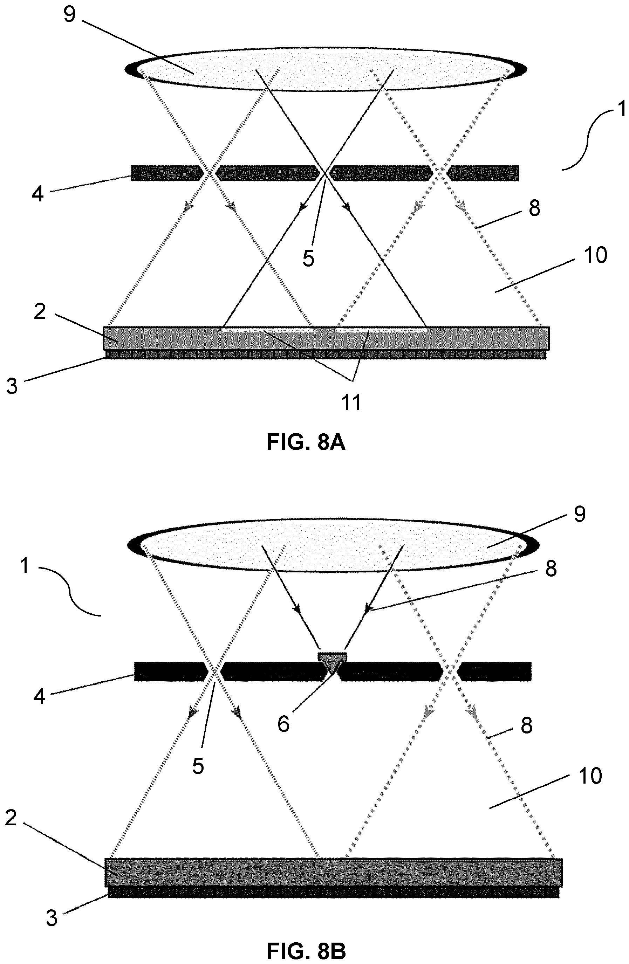

By way of operating example, Figures 8A-8B show two situations where, in one of them, the device (1) of the invention has all the pinholes (5) of the collimator (4) uncovered, and where in another, one of said pinholes (5) is covered with a plug (6). Preferably, the device (1) of the invention allows gamma rays (8) coming from a source (9) of radiation to be detected. Likewise, the aperture of each pinhole (5) defines a cone (10) of incidence. Additionally, as can be seen in Figure 8A, the cones (10) of incidence can have overlapping areas (11) with the cones (10) defined by the neighboring pinholes (5). In any of the preceding configurations, the variation of the position of the pinholes (5) of the collimator (4) with respect to the detector (2) takes place continuously, i.e., the pinholes (5) travel a relative distance with respect to the detector (2), but without occlusion of said pinholes along said distance, except possibly by plugs placed prior to acquisition.

In turn, as can be seen in Figure 8B, the pinholes (5) of the collimator (4) can be covered and/or moved independently, for the purpose of obtaining a general statistical sampling on the field of view (FOV) or to temporarily prevent the penetration of the gamma rays (8) in the overlapping area (11). With it and by means of a suitable processing of the detection data read by the electronics (3), it is possible to completely or partially eliminate the unwanted artifacts which produce the overlapping areas (11), as well as to obtain a complete image without truncation in the device.

The sensitive material of the detector (2) can be any radiation-sensitive material that produces a measurable physical quantity when radiation interacts with said material. Examples of detectors (2) usable in the scope of the invention are monolithic or pixelated scintillating crystals, organic or inorganic crystal scintillators, liquid scintillators and/or gaseous scintillators. The scintillators can produce a detection signal that is due to both scintillation and Cherenkov radiation processes. Organic crystal scintillators can be, for example, anthracene, stilbene, naphthalene, liquid scintillators (for example, organic liquids such as p-terphenyl (C 18H14), 2-(4-biphenylyl)-5-phenyl-1,3,4-oxadiazole PBD (C20H14N2O), butyl PBD (C24H22N2O), gas scintillators (such as nitrogen, helium, argon, krypton, xenon), inorganic crystal scintillators, or combinations of any of same. Commonly known inorganic scintillator crystals may be, for example, cesium iodide (Csl), thallium doped cesium iodide (Csl (Tl)), bismuth germanate (BGO), thallium doped sodium iodide (Nal (Tl)), barium fluoride (BaF2), europium doped calcium fluoride (CaF2(Eu)), cadmium tungstate (CdWO4), cerium doped lanthanum chloride (LaCb(Ce)), cerium doped yttrium lutetium silicates (LuYSiOs(Ce)(YAG(Ce)), silver doped zinc sulfide (ZnS(Ag)) or cerium doped yttrium aluminum garnet (III) Y3 Al5O 12 (Ce) or LYSO. Additional examples are CsF, KI(TI), CaF2(Eu), Gd2SiO5[Ce] (GSO), LSO, GAGG(Ce).

As mentioned, the scintillators can be monolithic crystals or pixelated crystals, or any combination thereof.

Scintillating photon detection devices may be formed by, for example, but not limited to, photosensors. The photosensors may be silicon photomultiplier arrays (SiPMs), single photon avalanche diodes (SPADs), digital SiPMs, avalanche photodiodes, position-sensitive photomultipliers, phototransistors, photo-ICs, or combinations thereof. This means that one detector (2) may be coupled, for example, to an SiPM array and another detector (2) in the same device (1) may be coupled to a phototransistor array, according to the above definitions.

Likewise, solid-state detectors (2) based on semiconductors such as Si, Ge, CdTe, GaAs, Pbl2, Hgl2, CZT, HgCdTe (also known as CTM), etc., and/or scintillation detectors (2) based on xenon and/or Cherenkov radiation detectors (2) based on PbF2, NaBi (WO4)2, PbWO4, MgF2, C6F14, C4F10 or silica aerogel, can be used.

Furthermore, the sensitive materials of the detector (2) can be encapsulated or exposed, coupled to an optical reflective surface and/or use any known technique to improve the quality of the collected data. The optical reflective surface can be polished or rough, specular, diffuse, retroreflective or composite. Likewise, one or more detectors (2) may comprise one or more optically painted surfaces.

Another object of the present invention relates to an imaging system by means of gamma ray detection, comprising one or more devices (1) according to any of the embodiments described herein. In said system, the electronic means (3) for the reading and processing of the detection signals of the gamma rays are preferably connected to an image reconstruction device from the processing of said signals.

In a preferred embodiment of the system of the invention, said system can be arranged in a mobile platform adapted for being oriented towards different regions of the source of gamma radiation.

## Claims

1. An imaging device (1) for imaging radiation-emitting objects (9) from the gamma ray detection (8) coming from said source (9) of radiation, comprising:
- a detector (2) equipped with one or more gamma radiation-sensitive materials;
- electronic means (3) configured for the reading and processing of one or more gamma radiation detection signals by the detector (1);
- a gamma ray collimator (4) equipped with a plurality of pinholes (5) which the gamma rays can penetrate, and arranged, relative to the detector (1), such that they provide at least one sampling region on the field of view (FOV) of the detector (2);
wherein the detector (2), the electronic reading and processing means (3) and the gamma ray collimator (4) of the device of the invention are arranged in substantially parallel respective planes, adopting a camera configuration with planar symmetry;
said device (1) being **characterized in that** the position of the pinholes (5) of said collimator (4) is variable relative to the position of the detector (2) in the device (1), such that the sampling region on the field of view (FOV) of the detector (2), provided by one or more cones (10) of incidence of gamma radiation upon passing through the pinholes (5), is modified with the variation of the positions of said pinholes (5) with respect to the detector (2); and wherein the collimator (4) and the detector (2) are arranged such that the variation of the position of the pinholes (5) of the collimator (4) with respect to the detector (2) takes place continuously, and such that the pinholes (5) travel a relative distance with respect to the detector (2), but without occlusion of said pinholes (5) along said distance during sampling.

2. The device (1) according to the preceding claim, wherein the positions of the pinholes (5) are independently movable with respect to one another in the collimator (4).

3. The device (1) according to any of the preceding claims, wherein the position of the collimator (4) and/or of its pinholes (5) exhibits relative rotational and/or translational movement with respect to the position of the detector (2).

4. The device (1) according to the preceding claim, wherein the position of the collimator (4) and/or of its pinholes (5) exhibits relative rotational movement with respect to the position of the detector (2), about an axis (4') substantially perpendicular to the plane formed by the collimator (4), where said axis (4') does not go through any of the pinholes of the collimator.

5. The device (1) according to the preceding claim, wherein the collimator (4) is mobile with respect to the detector (2), such that each of its pinholes (5) describes a circular path around its corresponding center of rotation, being different for each of the pinholes (5).

6. The device (1) according to the preceding claim, wherein said fixed point is such that the angles of visibility of the field of view (FOV) areas of the detector (2), under the movement of the collimator (4), are not repeated until a complete or partial rotation of said collimator (4) has been achieved.

7. The device (1) according to any of the preceding claims, wherein the axes of all the pinholes (5) of the collimator (4) are substantially parallel, such that the sampling region on the field of view of the detector (2) provided by a plurality of cones of incidence of gamma radiation upon passing through the pinholes (5) does not exhibit any overlap for at least two of said pinholes (5).

8. The device (1) according to any of the preceding claims, wherein one or more of the pinholes (5) of the collimator (4) are covered by corresponding plugs (6), the plugs being arranged on the collimator (4), or on a support (7) arranged thereon, wherein said support (7) is equipped with pinholes (5') adapted for housing the plugs (6).

9. The device (1) according to any of the preceding claims, having zero, one, or more overlapping areas between the cones (10) of incidence on at least one detection surface of the detector (2), and wherein said areas are modified with the variation of the positions of the pinholes (5) with respect to the detector (2).

10. The device (1) according to any of the preceding claims, comprising a plurality of detectors (2) arranged around the source of radiation (9), forming a closed or open ring structure, wherein the collimator (4) forms a structure coaxial to that of the detector (2).

11. The device (1) according to the preceding claim, wherein the structure formed by the collimator (4) is a rotating structure, or rotates and moves by translation describing a helical movement, relative to the structure formed by the detectors (2).

12. The device (1) according to any of the preceding claims, wherein the collimator (4) has:
- circular groove-shaped pinholes (5), and wherein said pinholes (5) are partially covered by a plug (6) with a variable position in said groove; and/or
- cylindrical-, wedge-, or double cone-shaped pinholes (5).

13. The device (1) according to any of the preceding claims, wherein the pinholes (5) of the collimator (4) are distributed such that each of said pinholes (5), except those arranged in the perimetral region of the collimator (4), has six neighboring pinholes (5), located at the same distance, forming a regular hexagon;
and wherein said pinholes (5) optionally have the same angular aperture.

14. An imaging system by means of gamma ray detection (8), comprising one or more devices (1) according to any of the preceding claims, wherein the electronic means (3) for the reading and processing of the signals of the detector (2) are connected to an image reconstruction device, from the processing of said signals.

15. The system according to the preceding claim, comprising a mobile platform adapted for orienting the device (1) towards different regions of a source (9) of gamma radiation.
